# EUROPEAN PATENT SPECIFICATION

(11) **EP 2 316 328 B1**
(45) Date of publication and mention of the grant of the patent: **09.05.2012**
(21) Application number: 10191689.8
(22) Date of filing: 14.09.2004
(51) Int. Cl.: A61B 1/04, A61B 1/005, A61B 19/00, A61B 1/018, A61B 1/267

(54) **Wrap-around holding device for use with bronchoscopes**
Umhüllungsvorrichtung zur Fixierung von Bronchoskopen
Dispositif de fixation à enroulement pour utilisation avec des bronchoscopes

(30) Priority: 15.09.2003 US 502615 P; 08.03.2004 US 550346 P; 26.04.2004 US 564944 P
(43) Date of publication of application: 04.05.2011
(62) Divisional of application: 09157586.0
(73) Proprietor: Super Dimension Ltd., Herzeliya 46120 (IL)
(72) Inventor: Greenburg, Benny, 45100, Hod Hasharon (IL); Belcher, Danny, 52504, Ramat Gan (IL); Griefner, Gil, 52531, Ramat Gan (IL); Becker, Heinrich, 46120, Herzeliya (IL)
(74) Representative: Modiano, Micaela Nadia

(56) References cited:
- WO-A2-02/24054
- US-A- 5 728 047

## Description

### FIELD AND BACKGROUND OF THE INVENTION

The present invention relates to bronchoscopy and, in particular, it concerns a system of accessories for use when performing surgical procedures using a bronchoscope.

The most common interventional procedure in the field of Pulmomary Medicine (i.e., medicine pertaining to the respiratory system) is bronchoscopy, in which a bronchoscope is inserted into the airways through the patient's nose or mouth. The instrument consists of a long, thin, flexible tube that typically contains three elements, an illumination arrangement for illuminating the region distal to the bronchoscope's tip via an optical fiber connected to an external light source, an imaging arrangement for delivering back a video image from the bronchoscope's distal tip, and a 'working channel' through which instruments of both diagnostic (e.g., biopsy tools) and therapeutic (e.g., laser, cryo or RF tissue elimination probes) nature are inserted. The distal tip of the bronchoscope is steerable; rotating a lever placed at the handle of the bronchoscope actuates the steering mechanism by deflection the tip in two opposite directions.

Bronchoscopies are applied routinely to the diagnosis and treatment of diseases such as Lung Cancer, Airway Stenosis, and Emphysema. They are performed by an expert pulmonologist, also known as a bronchoscopist.

Bronchoscopies are performed by a staff of at least two persons, the bronchoscopist and at least one assistant, usually a nurse. During a typical procedure, the bronchoscopist holds the bronchoscope handle with one hand and the bronchoscope tube with the other hand. He or she manipulates the distal tip of the bronchoscope inside the lung by rotating the deflection lever and by pushing and pulling the tube. Once the tip is brought to the target, a medical diagnosis is achieved and/or treatment is applied by insertion of a bronchoscopic tool into the working channel and out through the distal tip of the bronchoscope tube and performing the diagnosis or treatment.

During insertion and operation the bronchoscopic tool, the distal tip of the bronchoscope should be held steady at the target. Performing all of these tasks concurrently often requires three or four hands, two for securing the bronchoscope in place, and one to two more hands for inserting and actuating the bronchoscopic tool. The complexity of such multi-person operation, requiring delicate coordination between the physician and the assistant, often detracts from the resulting precision of the medical procedure, and the need for additional helping hands often increases its cost.

Of particular relevance to the present invention is a device and method described in PCT patent application publication no. WO 03/086498 entitled "Endoscope Structure and Techniques for Navigation in Brunched Structure" to Gilboa, which is hereby incorporated fully by reference. This patent application describes a method and apparatus in which a locatable guide ("LG"), enveloped by a sheath, is used to navigate a bronchoscopic tool to a location within the lung. The guide/sheath combination is inserted into the lung via the working channel of a bronchoscope. Once the tip of the guide is located at its target, a lock, which is placed at the orifice ("connection port") of the bronchoscope's working channel, is operated to prevent the sheath from sliding in or out of the bronchoscope. The guide is then withdrawn from the sheath, leaving the sheath in place to guide a tool to the required target location.

On the other hand, when the same bronchoscope is used in its primary function for investigation of the bronchi, the same working channel is used to clean disturbing mucus from the airways, using a vacuum pump connected to special connector separate from the orifice of the working channel. For the suction to work properly, the orifice of the working channel should be sealed during application of the suction.

It follows that, during the procedure, the physician typically needs to interchange two different devices to the connection port of the bronchoscope working channel, a seal and a lock, and these must be interchanged depending upon which function the bronchoscope is currently performing.

During the switch between locatable guide and other tools, it is necessary to insert the guide or tool into the free proximal end of the sheath. This step has been found to be somewhat "fiddly" and difficult to achieve quickly due to the flexibility and consequent mechanical instability of the end of the sheath. The problem can be addressed easily by holding the end of the sheath in one hand and the tool in another, but this would again require additional free hands during performance of the procedure.

In order to facilitate operation of a system such as described in the aforementioned application by a single practitioner, it would be preferable to allow the practitioner to temporarily release his or her grip on a secondary tool or device used via the working channel of the bronchoscope. At the same time, it is preferable that the device remains immediately accessible and operable, and does not hang loosely.

There is therefore a need for accessories for use with a bronchoscope which would facilitate operation of a bronchoscope and associated tools by a single practitioner. It would also be advantageous to provide an adapter for the connection port of the working channel of a bronchoscope which would perform both the sealing and tool-locking functions without requiring replacement of an attachment during the procedure. It would further be advantageous to provide an arrangement according to the teachings of the aforementioned PCT patent publication which would facilitate insertion of tools into the guide sheath.

### SUMMARY OF THE INVENTION

The present invention relates to a wrap-around handle extension for use with a bronchoscope handle, the wrap-around handle extension comprising: (a) a flexible wrap-around layer provided with complementary fastening arrangements deployed so as to form a conical sleeve for holding the bronchoscope handle; and (b) a hand loop associated with the flexible wrap-around layer and configured for receiving the hand of a user to allow suspension of the conical sleeve from the hand of the user.

According to a further feature of the present invention, the complementary fastening arrangements are implemented as complementary regions of a Velcro fastening arrangement.

According to a further feature of the present invention, there is also provided an accessory suspension strap associated with the flexible wrap-around layer and configured for suspending an accessory from the conical sleeve.

According to a further feature of the present invention, the accessory suspension strap is configured with a releasable fastening configuration to form a releasable suspension loop.

### BRIEF DESCRIPTION OF THE DRAWINGS

The invention is herein described, by way of example only, with reference to the accompanying drawings, wherein:
FIG. 1 is a side view of a wrap-around handle extension, constructed and operative according to the teachings of the present invention, in a flattened state;
FIG. 2 is a side view of the wrap-around handle extension of Figure 10 in a deployed state forming a conical sleeve for holding a bronchoscope handle;
FIG. 3 is a side view illustrating the use of the wrap-around handle extension of Figure 10 with a bronchoscope and an accessory during a first stage of a procedure; and
FIG. 4 is a side view illustrating the use of the wrap-around handle extension of Figure 10 with a bronchoscope and an accessory during a second stage of a procedure.

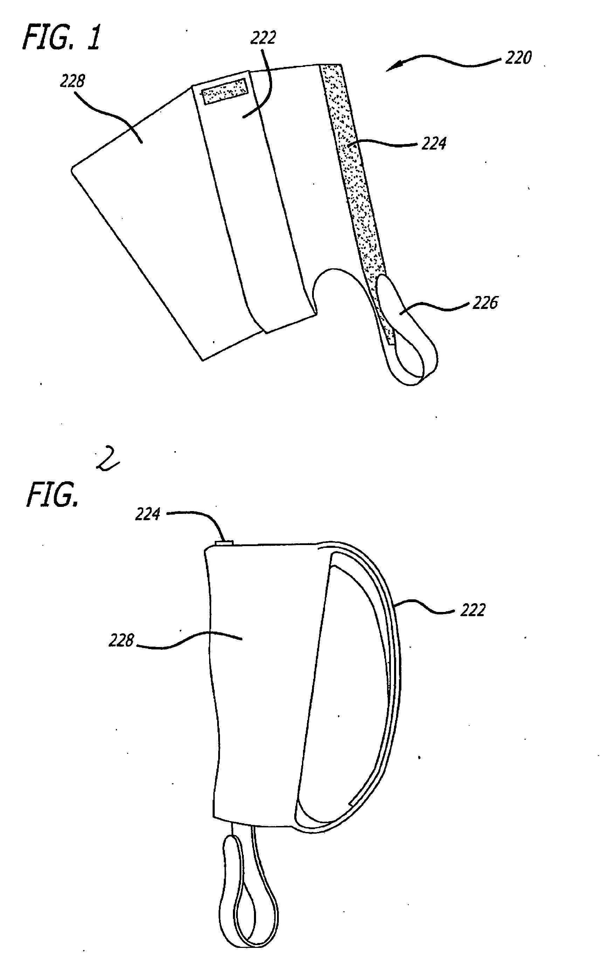

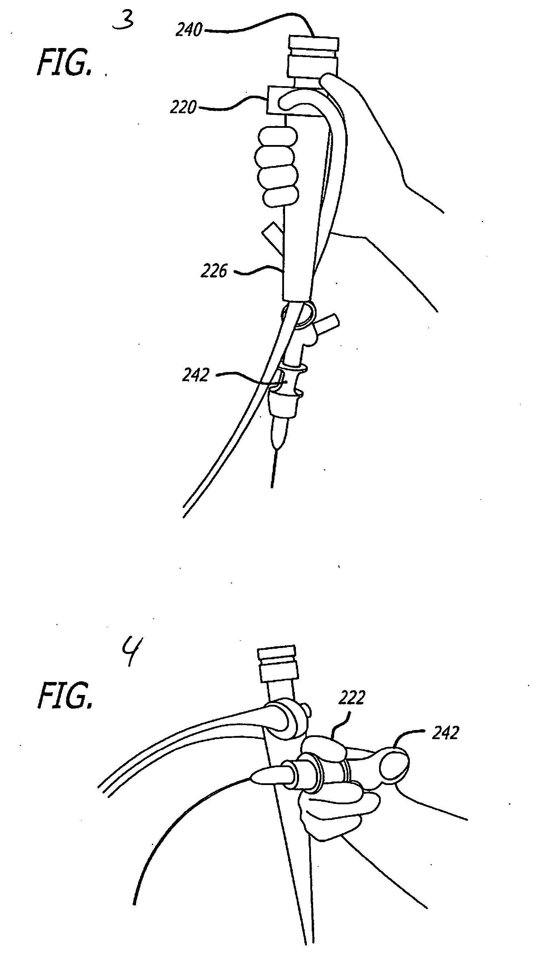

Figures 1-4 illustrated a wrap-around handle extension **220** for use with a bronchoscope handle. Generally speaking, wrap-around handle extension **220** helps the bronchoscopist in holding with one hand the bronchoscope's handle and concurrently the handle of the bronchoscope tool, and operating the two without them interfering with each other, while leaving the second hand free for any additional required action. To this end, handle extension **220** includes a flexible wrap-around layer **228** provided with complementary fastening arrangements **224** deployed so as to form a conical sleeve (Figure 2) for holding the bronchoscope handle as shown in Figures 3 and 4. Handle extension **220** also includes a hand loop **222** associated with the flexible wrap-around layer **228** and configured for receiving the hand of a user to allow suspension of the conical sleeve from the hand of the user.

Referring to the example illustrated herein in more detail, flexible wrap-around layer **228** is preferably implemented as a thin layer of soft plastic foam. Hand loop **222** is typically formed from similar foam material. Complementary fastening arrangements **224** are preferably implemented as complementary regions of a Velcro fastening arrangement, although other arrangements of straps or fasteners may be used.

Wrap-around handle extension **220** preferably also includes an accessory suspension strap **226** associated with flexible wrap-around layer **228** and configured for suspending an accessory from the conical sleeve (see Figure 3). Accessory suspension strap **226** is preferably configured with a releasable fastening configuration (for example Velcro) to form a releasable suspension loop. In a particularly preferred implementation as shown here, accessory suspension strap **226** is implemented as an extending tail of one of the Velcro straps of fastening arrangement **224.**

As mentioned before, wrap-around layer **228** forms a conical sleeve designed to wrap and fit to the handle of the bronchoscope **240.** When the bronchoscope in used for navigation, the bronchoscopic tool **242** is installed hanging in loop **226** as shown in Figure 3. This allows the bronchoscopist to manipulate the bronchoscope with both hands without being burdened by the bronchoscopic tool, although he is holding that tool as well. When the tip of the bronchoscope reaches to the target, the bronchoscopist put his palm inside the handle **222** so the bronchoscope hangs, or rests, on the back of his hand. Now the bronchoscopist may operate the bronchoscopic tool **242** using his free fingers, as shown in Figure 4.

The implementation illustrated herein is preferably a single-use disposable accessory. Other similar implementations, using different materials and different techniques for attaching the bronchoscopic tool to the bronchoscope's handle also fall within the scope of the present invention. In each case, the handle extension employs similar principles in that the handle of the tool is suspended in a position that does not interfere with the physician's use the bronchoscope for navigation inside the lungs, and yet after reaching to the target lets the physician operate the bronchoscopic tool using the same hand while still holding the bronchoscope's handle.

Although the above aspects of the present invention have been described in the context of a bronchoscope and bronchoscopic tools, it should be appreciated that other applications using other type of endoscopes and endoscopic tools, in which it is required to hold the tools and the endoscope handle at the same time, also fall within the scope of the present invention.

It will be appreciated that the above descriptions are intended only to serve as examples, and that many other embodiments are possible within the scope of the present invention as defined in the appended claims.

## Claims

1. A wrap-around handle extension for use with a bronchoscope handle, the wrap-around handle extension comprising:
(a) a flexible wrap-around layer provided with complementary fastening arrangements deployed so as to form a conical sleeve for holding the bronchoscope handle; and
(b) a hand loop associated with said flexible wrap-around layer and configured for receiving the hand of a user to allow suspension of said conical sleeve from the hand of the user.

2. The handle extension of claim 1, wherein said complementary fastening arrangements are implemented as complementary regions of a Velcro fastening arrangement.

3. The handle extension of claim 1, further comprising an accessory suspension strap associated with said flexible wrap-around layer and configured for suspending an accessory from said conical sleeve.

4. The handle extension of claim 3, wherein said accessory suspension strap is configured with a releasable fastening configuration to form a releasable suspension loop.

## Patentansprüche

1. Eine Umhüllungs-Griffverlängerung zur Verwendung mit einem Bronchoskopgriff, wobei die Umhüllungs-Griffverlängerung Folgendes umfasst:
(a) eine biegsame Umhüllungsschicht, ausgestattet mit komplementären Befestigungsanordnungen, bereitgestellt, um eine kegelförmige Hülse zum Halten des Bronchoskopgriffs zu bilden; und
(b) eine Handschlaufe, verbunden mit der biegsamen Umhüllungsschicht und ausgebildet, um die Hand eines Benutzers aufzunehmen, um die Aufhängung der kegelförmigen Hülse von der Hand des Benutzers zu ermöglichen.

2. Die Griffverlängerung gemäß Anspruch 1, wobei die komplementären Befestigungsanordnungen als komplementäre Bereiche einer Klettverschluss-Befestigungsanordnung ausgeführt sind.

3. Die Griffverlängerung gemäß Anspruch 1, die weiter einen Zusatzgerät-Aufhängungsriemen umfasst, verbunden mit der biegsamen Umhüllungsschicht und ausgebildet, um ein Zusatzgerät von der kegelförmigen Hülse aufzuhängen.

4. Die Griffverlängerung gemäß Anspruch 3, wobei der Zusatzgerät-Aufhängungsriemen mit einer lösbaren Befestigungsanordnung zum Bilden einer lösbaren Aufhängungsschlaufe ausgebildet ist.

## Revendications

1. Extension de poignée à enroulement, pour utilisation avec une poignée de bronchoscope, l'extension de poignée à enroulement comprenant :
(a) une couche à enroulement flexible, munie d'agencements de fixation complémentaires, déployés de manière à former une gaine conique pour maintenir la poignée de bronchoscope ; et
(b) une boucle à main, associée à ladite couche à enroulement flexible et configurée pour recevoir la main d'un utilisateur, pour permettre une mise en suspension de ladite gaine conique à partir de la main de l'utilisateur.

2. Extension de poignée selon la revendication 1, dans laquelle lesdits agencements de fixation complémentaires sont mis en oeuvre sous forme de régions complémentaires d'un agencement de fixation de type Velcro.

3. Extension de poignée selon la revendication 1, comprenant en outre une sangle de suspension d'accessoire, associée à ladite couche à enroulement flexible et configurée pour mettre en suspension un accessoire à partir de ladite gaine conique.

4. Extension de poignée selon la revendication 3, dans laquelle ladite sangle de suspension d'accessoire est configurée avec une configuration de fixation désolidarisable, de manière à former une boucle de suspension désolidarisable.
